# EUROPEAN PATENT APPLICATION

(11) **EP 4 631 522 A1**
(43) Date of publication of application: **15.10.2025**
(21) Application number: 23900086.2
(22) Date of filing: 08.12.2023
(51) Int. Cl.: A61K 39/395, A61K 9/19, A61K 47/12, A61K 47/26, C07K 16/24, A61P 19/02, A61P 19/08, A61P 1/00, A61P 3/10, A61P 17/06, A61P 29/00, A61P 37/02, A61P 25/00

(54) **IL-17A ANTIBODY FORMULATION AND USE THEREOF**

(30) Priority: 09.12.2022 CN 202211576141
(71) Applicant: Shanghai Huaota Biopharmaceutical Co., Ltd., Shanghai 201203 (CN); Huabo Biopharm (Shanghai) Co., Ltd., Shanghai 201203 (CN)
(72) Inventor: JIA, Huifeng, Shanghai 201203 (CN); PAN, Xianfei, Shanghai 201203 (CN); WANG, Shaojie, Shanghai 201203 (CN); CUI, Xiaopei, Shanghai 201203 (CN); ZHU, Xiangyang, Shanghai 201203 (CN)
(74) Representative: karo IP
(86) International application number: PCT/CN2023/137417
(87) International publication number: WO 2024/120518

(57) **Abstract**

The present application relates to a pharmaceutical composition of an anti-human IL-17A monoclonal antibody and use thereof. The pharmaceutical composition comprises: (a) an anti-human IL-17A monoclonal antibody; and (b) a pharmaceutically acceptable carrier. The pharmaceutical composition described in the present application can improve the stability of the anti-human IL-17A monoclonal antibody, thus reducing the administration frequency, and improving the safety and effectiveness of clinical use.

## Description

### TECHNCIAL FIELD

The present application relates to the field of biomedicine, in particular to a pharmaceutical composition of an IL-17A antibody.

### BACKGROUND ART

IL-17A (Interleukin 17A) is a homodimer composed of two chains of 155 amino acids connected by disulfide bonds, with a molecular weight of 35 kDa. It was originally discovered to be secreted by activated CD4+ T cells. This subset of T cells that characteristically secretes IL-17A is called Th17 cells. In addition to Th17 cells, cytotoxic CD8+ T cells (Tc17), γδ T cells, natural killer T cells (NKT-17) and B cells can also express IL-17A under specific conditions. IL-17A can also be produced by innate immune cells, including monocytes, neutrophils, natural killer cells, and lymphoid tissue-inducing-like (Lti-like) cells.

IL-17-bound type I cell surface receptors are called IL-17Rs, of which there are at least three types: IL-17RA, IL-17RB, and IL-17RC. IL-17A and IL-17F bind to IL-17RA and IL-17RC receptor complexes in the form of homodimers or heterodimers to transduce signals and are involved in the body's autoimmune diseases, various inflammatory responses, and host anti-infection immune responses.

IL-17A mainly induces signaling activation in non-hematopoietic cells, including epithelial cells and stromal cells. IL-17A-induced expression of a variety of inflammatory factors and chemokines can promote the recruitment of a variety of immune cells, thereby promoting autoimmune diseases. Studies have found that IL-17A and IL-17F could promote the inflammatory response by inducing the expression of several inflammatory and chemokines in the target cells. IL-17A binds to cell surface receptor IL-17RA, recruits IL-17RC to form heterodimers, mediates downstream signaling pathways, and also plays an important role in a variety of autoimmune diseases, including autoimmune diseases such as rheumatoid arthritis (RA) and multiple sclerosis (MS), as well as inflammatory bowel disease (IBD), psoriasis, systemic lupus erythematosus (SLE) and type 1 diabetes (T1D).

Anti-human IL-17A monoclonal antibodies (such as humanized anti-human IL-17A monoclonal antibodies) can prevent and treat IL-17A-mediated diseases. Among others, humanized anti-human IL-17A monoclonal antibodies are macromolecular drugs with complex structures, and during the production, storage and transportation of the drugs, they are affected by various physical and chemical factors and will undergo aggregation, hydrolysis, oxidation and other reactions. The by-products produced can have adverse effects on the safety and efficacy of the drugs, so it is very important to develop formulation formulas with excellent stability for clinical medication. At the same time, multiple medications also bring great inconvenience to patients.

Therefore, there is a need in the art to develop an IL-17A monoclonal antibody pharmaceutical formulation with excellent stability to improve the stability of the IL-17A monoclonal antibody and better meet the clinical medication needs.

### SUMMARY OF THE INVENTION

The purpose of the present application is to provide a stable IL-17A antibody pharmaceutical composition, and the pharmaceutical composition can maintain excellent stability of the IL-17A antibody. At the same time, the IL-17A antibody in the pharmaceutical composition can be stably present at a high concentration, which can not only ensure the safety of clinical medication, but also effectively reduce the frequency of medication for patients and improve the effectiveness of clinical medication.

In an aspect, the present application provides a pharmaceutical composition, comprising: an IL-17A antibody, and optionally a pharmaceutically acceptable carrier, wherein the concentration of the IL-17A antibody in the pharmaceutical composition is 125-155 mg/mL.

In some embodiments, the IL-17A antibody is an anti-human IL-17A antibody.

In some embodiments, the anti-human IL-17A antibody is a humanized anti-human IL-17A antibody.

In some embodiments, the IL-17A antibody comprises three complementary determining regions HCDR1, HCDR2 and HCDR3 of an antibody heavy chain variable region and three complementary determining regions LCDR1, LCDR2 and LCDR3 of an antibody light chain variable region, the HCDR1 comprises an amino acid sequence as set forth in SEQ ID NO: 1, the HCDR2 comprises an amino acid sequence as set forth in SEQ ID NO: 2, the HCDR3 comprises an amino acid sequence as set forth in SEQ ID NO: 3, the LCDR1 comprises an amino acid sequence as set forth in SEQ ID NO: 4, the LCDR2 comprises an amino acid sequence as set forth in SEQ ID NO: 5 (KVS), and the LCDR3 comprises an amino acid sequence as set forth in SEQ ID NO: 6.

In some embodiments, the heavy chain variable region VH of the IL-17A antibody comprises an amino acid sequence as set forth in SEQ ID NO: 7, and the light chain variable region of the IL-17A antibody comprises an amino acid sequence as set forth in SEQ ID NO: 8.

In some embodiments, the concentration of the IL-17A antibody is 130 mg/mL.

In some embodiments, the concentration of the IL-17A antibody is 150 mg/mL.

In some embodiments, the dosage form of the pharmaceutical composition is a liquid formulation.

In some embodiments, the dosage form of the pharmaceutical composition is an injection formulation or an infusion formulation.

In some embodiments, the pharmaceutically acceptable carrier in the pharmaceutical composition comprises a buffer, a stabilizer and/or a surfactant.

In some embodiments, the buffer is selected from the group consisting of acetate-acetic acid buffer, citrate-citric acid buffer, phosphate buffer, histidine-histidine hydrochloride buffer, histidine-acetic acid buffer, tris(hydroxymethyl)aminomethane (Tris)-hydrochloride buffer, phosphoric acid-citrate salt, or a combination thereof.

In some embodiments, the buffer is selected from the group consisting of acetate-acetic acid buffer, histidine-histidine hydrochloride buffer, histidine-acetic acid buffer, tris(hydroxymethyl)aminomethane (Tris)-hydrochloride buffer, phosphoric acid-citrate salt, or a combination thereof.

In some embodiments, the concentration of the buffer is 5-100mM.

In some embodiments, the concentration of the buffer is 10-80mM.

In some embodiments, the concentration of the buffer is 10-50mM.

In some embodiments, the solvent of the buffer is water.

In some embodiments, the stabilizer is selected from the group consisting of sodium chloride, amino acid, sugar alcohol, or a combination thereof.

In some embodiments, the amino acid is one or more selected from the group consisting of proline, arginine, glycine, histidine, methionine, or a combination thereof.

In some embodiments, the sugar alcohol is selected from the group consisting of sucrose, mannitol, trehalose, maltose, sorbitol, or a combination thereof.

In some embodiments, the stabilizer is proline, histidine, arginine, sucrose, trehalose, mannitol, or a combination thereof.

In some embodiments, the concentration of the sodium chloride is 50-400mM.

In some embodiments, the concentration of the sodium chloride is 100-300mM.

In some embodiments, the concentration of the sodium chloride is 150-300mM.

In some embodiments, the concentration of the amino acid is 10-300mM.

In some embodiments, the concentration of the amino acid is 30-180mM.

In some embodiments, the concentration of the amino acid is 50-150mM.

In some embodiments, the concentration of the sugar alcohol is 0.1-20 wt.%, based on the total weight of the pharmaceutical composition.

In some embodiments, the concentration of the sugar alcohol is 2-16 wt.%, based on the total weight of the pharmaceutical composition.

In some embodiments, the concentration of the sugar alcohol is 2-14 wt.%, based on the total weight of the pharmaceutical composition.

In some embodiments, the concentration of the sugar alcohol is 4-12 wt.%, based on the total weight of the pharmaceutical composition.

In some embodiments, the content of the stabilizer is 0.1-20 wt.%, based on the total weight of the pharmaceutical composition.

In some embodiments, the content of the stabilizer is 2-16 wt.%, based on the total weight of the pharmaceutical composition.

In some embodiments, the content of the stabilizer is 2-14 wt.%, based on the total weight of the pharmaceutical composition.

In some embodiments, the content of the stabilizer is 4-12 wt.%, based on the total weight of the pharmaceutical composition.

In some embodiments, the concentration of the stabilizer is 10-200g/L.

In some embodiments, the concentration of the stabilizer is 20-160g/L.

In some embodiments, the concentration of the stabilizer is 20-140g/L.

In some embodiments, the concentration of the stabilizer is 40-120g/L.

In some embodiments, the surfactant is selected from the group consisting of polyoxyethylene sorbitan fatty acid ester, polyoxyethylene hydrogenated castor oil, glycerol fatty acid ester, polysorbate, poloxamer, or a combination thereof.

In some embodiments, the polysorbate is selected from the group consisting of polysorbate 20 (PS-20, Tween-20), polysorbate 40 (PS-40), polysorbate 60 (PS-60), and polysorbate 80 (PS-80).

In some embodiments, the poloxamer is selected from the group consisting of poloxamer 188, poloxamer 108, poloxamer 124, or a combination thereof.

In some embodiments, the surfactant is polysorbate.

In some embodiments, the content of the surfactant is 0.001-0.2 wt.%, based on the total weight of the pharmaceutical composition.

In some embodiments, the content of the surfactant is 0.001-0.1 wt.%, based on the total weight of the pharmaceutical composition.

In some embodiments, the content of the surfactant is 0.005-0.05 wt.%, based on the total weight of the pharmaceutical composition.

In some embodiments, the pharmaceutically acceptable carrier in the pharmaceutical composition also includes a chelator.

In some embodiments, the chelator is selected from the group consist of disodium ethylene diamine tetraacetic acid (EDTA), diethylene triamine pentaacetic acid (DTPA), or a combination thereof.

In some embodiments, the content of the chelator is 0.001-0.01 wt.%, based on the total weight of the pharmaceutical composition.

In some embodiments, the pH range of the pharmaceutical composition is 4.5-7.2.

In some embodiments, the pH range of the pharmaceutical composition is 4.7-6.5.

In some embodiments, the pH range of the pharmaceutical composition is 4.7-6.0.

In some embodiments, the buffer in the pharmaceutical composition is acetate-acetic acid buffer, and the pharmaceutical composition has a pH of 4.7-6.0.

In some embodiments, the buffer in the pharmaceutical composition is histidine-acetic acid buffer, and the pharmaceutical composition has a pH of 4.7-6.0.

In some embodiments, the pharmaceutical composition comprises 20mM sodium acetate-acetic acid buffer, 7 wt.% sucrose, 0.03 wt.% Tween-80, pH5.2±0.3, and anti-human IL-17A monoclonal antibody of 150mg/mL.

In some embodiments, the pharmaceutical composition comprises 20mM histidine-acetic acid buffer, 7.5wt.% sucrose, 0.03 wt.% Tween-80, pH5.4±0.3, and anti-human IL-17A monoclonal antibody of 150mg/mL.

In another aspect, the present application provides a lyophilized formulation comprising the pharmaceutical composition.

In some embodiments, the lyophilized formulation is prepared by freeze-drying the pharmaceutical composition to obtain the lyophilized formulation.

In some embodiments, the lyophilized formulation further comprises a lyoprotectant.

In some embodiments, the lyoprotectant is selected from the group consisting of sucrose, mannitol, trehalose, maltose, sorbitol, glucose, fructose, galactose, or a combination thereof.

In another aspect, the present application further provides a kit comprising the pharmaceutical composition, or the lyophilized formulation, and a container for containing the pharmaceutical composition or the lyophilized formulation.

In some embodiments, the kit further comprises instructions for use of the pharmaceutical composition or the lyophilized formulation.

In another aspect, the present application further provides use of the pharmaceutical composition, the lyophilized formulation or the kit in the preparation of a medicament for preventing and/or treating IL-17A-mediated diseases and/or disorders.

In some embodiments, the IL-17A-mediated diseases and/or disorders include autoimmune diseases.

In another aspect, the present application further provides use of the pharmaceutical composition, the lyophilized formulation or the kit in the preparation of a medicament for preventing and/or treating autoimmune diseases and/or disorders.

In some embodiments, the disease and/or disorder is mediated by IL-17A.

In some embodiments, the autoimmune disease is selected from the group consisting of psoriasis, arthritis, multiple sclerosis, ankylosing spondylitis, inflammatory bowel disease, systemic lupus erythematosus, type I diabetes, or a combination thereof.

In some embodiments, that arthritis is rheumatoid arthritis.

In another aspect, the present application further provides a method for preventing and/or treating IL-17A-mediated diseases and/or disorders, comprising administering the pharmaceutical composition, the lyophilized formulation or the kit to a subject in need thereof.

In some embodiments, the IL-17A-mediated diseases and/or disorders include autoimmune diseases.

In some embodiments, the autoimmune disease is selected from the group consisting of psoriasis, arthritis, multiple sclerosis, ankylosing spondylitis, inflammatory bowel disease, systemic lupus erythematosus, type I diabetes, or a combination thereof.

In another aspect, the present application further provides the pharmaceutical composition, the lyophilized formulation or the kit for use in preventing and/or treating IL-17A-mediated diseases and/or disorders.

In some embodiments, the IL-17A-mediated diseases and/or disorders include autoimmune diseases.

In some embodiments, the autoimmune disease is selected from the group consisting of psoriasis, arthritis, multiple sclerosis, ankylosing spondylitis, inflammatory bowel disease, systemic lupus erythematosus, type I diabetes, or a combination thereof.

The present application mainly has the following beneficial effects:
1. The present application provides a pharmaceutical composition containing an anti-human IL-17A monoclonal antibody (such as a humanized anti-human IL-17A monoclonal antibody), which can significantly enhance the stability of the anti-human IL-17A monoclonal antibody and maintain its stability under stress (high temperature, freezing-thawing and shaking, etc.), accelerated and long-term cold storage conditions.
2. The pharmaceutical composition containing an anti-human IL-17A monoclonal antibody (such as a humanized anti-human IL-17A monoclonal antibody) of the present application can enhance the stability of high-concentration (greater than 150mg/mL) anti-human IL-17A monoclonal antibodies.
3. The pharmaceutical composition containing an anti-human IL-17A monoclonal antibody (such as a humanized anti-human IL-17A monoclonal antibody) of the present application can enhance the thermodynamic and chemical stability of the antibody formulation, so that the antibody can be stably stored in the new formulation, which improves product quality while extending the shelf life of the product and improving clinical safety.
4. The present application effectively increases the concentration of anti-human IL-17A antibodies in the pharmaceutical composition, can effectively reduce the frequency of administration for subjects, improve the effectiveness of clinical medication, save medication procedures, and improve medication efficiency.

Those skilled in the art can easily discern other aspects and advantages of the present application from the following detailed description. Only exemplary embodiments of the present application are shown and described in the following detailed description. As those skilled in the art will realize, the contents of the present application enable those skilled in the art to make changes to the specific embodiments disclosed without departing from the spirit and scope of the invention covered by the present application. Accordingly, the drawings and descriptions in the specification of the present application are illustrative only and not restrictive.

### BRIEF DESCRIPTION OF DRAWINGS

The specific features of the invention to which the present application relates are set forth in the appended claims. The features and advantages of the invention to which the present application relates can be better understood by reference to the exemplary embodiments described in detail below and the drawings. The accompanying drawings are briefly described as follows:
Fig. 1 shows the flow state of high-concentration samples of the present application when stored upright at low temperature.
Fig. 2 shows the flow state of high-concentration samples of the present application when stored inverted at low temperature.

### DETAILED DESCRIPTION OF THE INVENTION

The embodiments of the invention of the present application will be described below with specific examples. One skilled in the art can easily understand other advantages and effects of the invention of the present application from the disclosure of the specification.

### Definition of Terms

In the present application, the term "IL-17A antibody" generally refers to an antibody targeting IL-17A, and the antibody may include any antibody form or an antigen-binding fragment thereof known in the art. For example, the antibody form may include, but is not limited to, monoclonal antibodies, chimeric antibodies, humanized antibodies and the like. For example, the antigen-binding fragment may include, but is not limited to, a Fab fragment, a Fab' fragment, a F(ab')₂ fragment, or a single Fv fragment, etc. having antigen-binding activity.

In the present application, the terms "stability" and "stable" generally refer to the fact that the active molecules in the antibody-containing formulation do not or only rarely aggregate, degrade or fragment under given production, preparation, transportation and/or storage conditions. A "stable" liquid formulation retains biological activity under given production, preparation, transportation and/or storage conditions. The stability of the antibody can be evaluated by measuring the aggregation, degradation or fragmentation degree of the formulation, for example, by size exclusion high performance liquid chromatography (SE-HPLC), reducing capillary electrophoresis sodium dodecyl sulphate (rCE-SDS), non-reducing capillary electrophoresis sodium dodecyl sulphate (nrCE-SDS), imaged capillary isoelectric focusing (icIEF) and/or particle size (PM) detection technology.

In the present application, the term "saccharide" may generally include different amounts of saccharide units, such as monosaccharides, disaccharides, trisaccharides, tetrasaccharides, pentasaccharides and polysaccharides. Saccharides also include their derivatives, such as amino sugars, sugar alcohols, and aldoses, etc. Examples of saccharides may include glucose, fructose, galactose, mannose, sorbose, ribose, deoxyribose, sucrose, trehalose, lactose, maltose, maltodextrin, dextran, cyclodextrin, and/or starch.

In the present application, the term "polysorbate" generally refers to oleic acid esters of sorbitol and its anhydrates, for example, copolymerized with ethylene oxide. Polysorbate may include polysorbate 20 (polyethylene oxide (20) sorbitan monolaurate, Tween 20, or PS20), and polysorbate 80 (polyethylene oxide (80) sorbitan monolaurate, Tween 80 or PS80).

In the present application, the term "w/w" generally refers to "mass to volume ratio", which is the ratio of the mass of a component to the volume of a formulation.

In the present application, the term "prevent" refers to a method of preventing the onset of a disease and/or its attendant symptoms or protecting a subject from acquiring a disease. As used herein, "prevent" also includes delaying the onset of a disease and/or its attendant symptoms and reducing a subject's risk of developing a disease.

In the present application, the term "treat" refers to any treatment of a disease in a mammal, including (but not limited to): (a) inhibiting the disease, that is, slowing or arresting the development of clinical symptoms; and/or (b) relieving the disease, that is, causing regression of clinical symptoms, and/or (c) alleviating or eliminating the disease and/or its attendant symptoms.

In the present application, the terms "comprise", "include", and "contain" are used interchangeably and include not only closed definitions but also semi-closed and open definitions. In other words, the term includes "consist of," "consist essentially of."

In the present application, "mM" means mmol/L unit, for example, 1 mM=1 mmol/L.

In the pharmaceutical composition of the present application, the weight content (wt.%) or concentration (such as mM, mg/mL) of the components is based on the weight or volume of the pharmaceutical composition.

In the present application, tris(hydroxymethyl)aminomethane is referred to as Tris for short, that is, "tris(hydroxymethyl)aminomethane" and "Tris" can be used interchangeably.

In the present application, the term "about" typically refers to a change in a range of 0.5%-10% greater or less than a specified value, e.g., a change in a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% greater or less than the specified value.

In the present application, the term "and/or" shall be understood to mean any one or two or more of the options.

### Detailed Description of the Invention

### Pharmaceutical composition

In an aspect, the present application provides a pharmaceutical composition comprising:
(a) an anti-human IL-17A antibody; and
(b) a pharmaceutically acceptable carrier.

In the present application, the pharmaceutically acceptable carrier may include a buffer, a stabilizer and a surfactant.

The term "pharmaceutically acceptable carrier" refers to one or more compatible solid, semisolid, liquid or gel fillers that are suitable for human or animal use and must be sufficiently pure and sufficiently low in toxicity. "Compatibility" refers to the ability of the components in a pharmaceutical composition and the active ingredients of a drug to blend with each other without significantly reducing the efficacy of the drug.

In the present application, the pH range of the pharmaceutical composition is about 4.5±0.3-7.2±0.3. For example, the pH range of the pharmaceutical composition is about 4.7±0.3-6.5±0.3. For example, the pH range of the pharmaceutical composition is about 4.7±0.3-6.0±0.3. For example, the pharmaceutical composition has a pH range of about 4.7±0.3, about 4.8±0.3, about 4.9±0.3, about 5.0±0.3, about 5.1±0.3, about 5.2±0.3, about 5.3±0.3, about 5.4±0.3, about 5.5±0.3, about 5.6±0.3, about 5.7±0.3, about 5.8±0.3, about 5.9±0.3, about 6.0±0.3, about 6.1±0.3, about 6.2±0.3, about 6.3±0.3, about 6.4±0.3, or about 6.5±0.3.

In the present application, the dosage form of the pharmaceutical composition may be a liquid formulation. For example, the dosage form of the pharmaceutical composition is an injection formulation or an infusion formulation.

### IL-17A antibody

In the present application, the active ingredient of the pharmaceutical composition may be an IL-17A antibody. For example, the antibody may be a monoclonal antibody. For example, the antibody may be a humanized antibody. For example, the antibody may be an anti-human IL-17A antibody.

In the present application, the IL-17A antibody may be a humanized anti-human IL-17A monoclonal antibody. In a certain embodiment, the humanized anti-human IL-17A monoclonal antibody (see CN108359011A); patent title: Antibody Targeting Interleukin 17A, Preparation Method and Use Thereof, has, in its amino acid sequence, HCDR1, HCDR2 and HCDR3 amino acid sequences of SEQ ID NO.: 1, SEQ ID NO.: 2 and SEQ ID NO.: 3, respectively, and LCDR1, LCDR2 and LCDR3 amino acid sequences of SEQ ID NO.: 4, SEQ ID NO.: 5 (KVS) and SEQ ID NO.: 6, respectively, wherein the sequence of the antibody CDR is classified according to the IMGT classification scheme, and the amino acid sequences are shown as follows:

| Domain | | Anti-IL-17A antibody | |
|---|---|---|---|
| | | Sequence | SEQ ID NO |
| VH | HCDR1 | EYIFTNY | 1 |
| | HCDR2 | DTNTGE | 2 |
| | HCDR3 | ANYGWGYFDY | 3 |
| VL | LCDR1 | QSLVHSNGYTY | 4 |
| | LCDR2 | KVS | 5 |
| | LCDR3 | SQSTHVPYT | 6 |

In the present application, the humanized anti-human IL-17A monoclonal antibody may have two amino acid sequences, namely, the heavy chain variable region SEQ ID NO.: 7 and the light chain variable region SEQ ID NO.: 8.
SEQ ID NO.: 7 
QVQLVQSGSELKKPGASVKVSCKASEYIFTNYGMNWVKQAPGQGFEWMGWIDT
NTGEPTYAQGFTGRFVFSLDTSVSTAYLQISSLKAEDTATYYCANYGWGYFDYWGQ
GTTVTVSS
 where the underscored are HCDR1, HCDR2, HCDR3 (SEQ ID NO.: 1, 2, and 3).
SEQ ID NO.: 8 
DVVMTQTPPSLPVNPGEPASISCRSSQSLVHSNGYTYLHWYLQKPGQSPQLLIYK
VSNHLSGVPDRFSGSGSGTDFTLKISWVEAEDVGVYFCSQSTHVPYTFGGGTKLEIK
 where the underscored are LCDR1, LCDR2, LCDR3 (SEQ ID NO.: 4, 5, and 6).

In the present application, the heavy chain variable region of the IL-17A antibody may comprise an amino acid sequence having at least 70% (e.g. at least 75%, 80%, 85%, 90%, 95%, 96%, 97%, 98%, 99% or more) sequence identity with the amino acid sequence as set forth in SEQ ID NO: 7.

In the present application, the IL-17A antibody may further comprise a heavy chain constant region. For example, the heavy chain constant region may be derived from the heavy chain constant region of IgG. For example, the IgG may be a human IgG. For example, the IgG may be IgG1, IgG2, IgG3 or IgG4. For example, the antibody heavy chain constant region of the IL-17A comprises an amino acid sequence as set forth in SEQ ID NO: 9.

In the present application, the light chain variable region of the IL-17A antibody may comprise an amino acid sequence having at least 70% (e.g. at least 75%, 80%, 85%, 90%, 95%, 96%, 97%, 98%, 99% or more) sequence identity with the amino acid sequence as set forth in SEQ ID NO: 8.

In the present application, the IL-17A antibody may further comprise a light chain constant region. For example, the light chain constant region is derived from Igκ. For example, the light chain constant region of the IL-17A comprises an amino acid sequence as set forth in SEQ ID NO: 10.

In the present application, the concentration of the IL-17A antibody is 125-155mg/mL, for example, the concentration of the IL-17A antibody is about 125mg/mL, about 130mg/mL, about 135mg/mL, about 140mg/mL, about 145mg/mL, about 150mg/mL or about 155mg/mL.

### Buffer

The pharmaceutical composition of the present application may comprise a buffer component. In the present application, the buffer includes (but is not limited to) acetate-acetic acid buffer, citrate-citric acid buffer, phosphate buffer, histidine-histidine hydrochloride buffer, histidine-acetic acid buffer, tris(hydroxymethyl)aminomethane (Tris)-hydrochloride buffer, phosphoric acid-citrate salt, or a combination thereof. For example, the buffer may consist of one or more of the above components.

In some specific embodiments, the buffer is selected from the group consisting of acetate-acetic acid buffer, histidine-histidine hydrochloride buffer, histidine-acetic acid buffer, tris(hydroxymethyl)aminomethane (Tris)-hydrochloride buffer, phosphoric acid-citrate salt, or a combination thereof.

It should be noted that, in the present application, reference to a formulation containing a buffer or a buffer system also includes reference to a formulation containing a buffering agent through which a buffer system is formed in the formulation.

For example, the buffer of the pharmaceutical composition may be sodium acetate-acetic acid buffer.

For example, the buffer of the pharmaceutical composition may be histidine-acetic acid buffer.

For example, the buffer of the pharmaceutical composition may be histidine-histidine hydrochloride buffer.

In the present application, the concentration of the buffer is about 5-100mM. For example, the concentration of the buffer is about 10-80mM. For example, the concentration of the buffer is about 10-50mM. For example, the concentration of the buffer is about 1mM-45mM, about 1mM-40mM, about 1mM-35mM, about 1mM-30mM, about 1mM-25mM, about 1mM-20mM, about 1mM-15mM, about 2mM-15mM, about 2mM-20mM, or about 5mM-20mM. For example, the concentration of the buffer in the pharmaceutical composition may be about 20mM. For example, the concentration of the buffer in the pharmaceutical composition may be about 40mM.

In the present application, the solvent of the buffer may be water for conventional use.

### Stabilizer

In the present application, the pharmaceutical composition may further comprise one or more suitable stabilizers. For example, the stabilizer may be a pharmaceutical excipient that protects the pharmaceutically active ingredient (such as the IL-17A antibody) from chemical and/or physical degradation during manufacture, storage and/or use. Due to the large molecular weight of protein, amino acids are more likely to degrade. Various physical and chemical reactions can cause amino acids to undergo changes such as deamination, cyclization, and chemical bond breakage. In some cases, the stabilizers include, but are not limited to, suitable saccharides, amino acids, polyols, or suitable derivatives or mixtures thereof.

In the pharmaceutical composition of the present application, the stabilizer includes (but is not limited to): sodium chloride, amino acid, sugar alcohol, or a combination thereof.

For example, the amino acid includes (but is not limited to): proline, arginine, glycine, histidine, methionine, or a combination thereof and pharmaceutically acceptable salts thereof. The amino acid may be present in the form of an amino acid salt, for example, an amino acid hydrochloride. For example, proline may be added to the pharmaceutical composition.

In the present application, the concentration of the amino acid may be 10-300mM. For example, the concentration of the amino acid may be about 30-180mM. For example, the concentration of the amino acid may be about 50-150mM. For example, the concentration of the amino acid may be about 10mM-300mM, about 50mM-300mM, about 50mM-250mM, about 50mM-200mM, or about 100mM-200mM. For example, the concentration of the amino acid may be about 10mM, about 20mM, about 50mM, about 70mM, about 90mM, about 100mM, about 120mM, about 150mM, about 180mM, about 200mM, about 250mM, about 270mM or about 300mM.

In the present application, the stabilizer that can be used in the pharmaceutical composition may include saccharides. Suitable saccharides may include, but are not limited to, amino sugars, sugar alcohols, aldoses, and the like. Examples of saccharides may include glucose, fructose, galactose, mannose, sorbose, ribose, deoxyribose, sucrose, trehalose, lactose, maltose, maltodextrin, dextran, cyclodextrin and starch, or a suitable mixture thereof. For example, the saccharide may include one or more selected from the group consisting of sucrose, trehalose and cyclodextrin. For example, the saccharide may include one or more selected from the group consisting of sucrose and trehalose. For example, the saccharide may include sucrose.

For example, the sugar alcohol includes (but is not limited to): sucrose, mannitol, trehalose, maltose, sorbitol, or a combination thereof.

For example, the stabilizer includes (but is not limited to): proline, histidine, arginine, sucrose, trehalose, mannitol, or a combination thereof.

In the present application, the concentration of the sugar alcohol may be 0.1-20 wt.%. For example, the concentration of the sugar alcohol may be 2-16 wt.%. For example, the concentration of the sugar alcohol may be 2-14 wt.%. For example, the concentration of the sugar alcohol may be 4-12 wt.%. For example, the concentration of the sugar alcohol may be about 0.1%, about 0.5%, about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, or about 20%. In the present application, the concentration unit of the sugar alcohol is based on the total weight of the pharmaceutical composition.

In the present application, the concentration of the sodium chloride is about 50-400mM. For example, the concentration of the sodium chloride may be about 100-300mM. For example, the concentration of the sodium chloride may be about 150-300mM. For example, the concentration of the sodium chloride may be about 50mM, about 70mM, about 90mM, about 100mM, about 120mM, about 150mM, about 180mM, about 200mM, about 250mM, about 270mM, about 300mM, about 350mM, or about 400mM.

In the present application, the content of the stabilizer is about 0.1-20 wt.%. For example, the content of the stabilizer is about 2-16 wt.%. For example, the content of the stabilizer is about 2-14 wt.%. In the present application, the content of the stabilizer is about 4-12 wt.%. In the present application, the content is based on the total weight of the pharmaceutical composition. For example, the content of the stabilizer can be about 0.1wt.%, about 0.5wt.%, about 1wt.%, about 2wt.%, about 3wt.%, about 4wt.%, about 5wt.%, about 6wt.%, about 7wt.%, about 8wt.%, about 9wt.%, about 10wt.%, about 11wt.%, about 12wt.%, about 13wt.%, about 14wt.%, about 15wt.%, about 16wt.%, about 17wt.%, about 18wt.%, about 19wt.% or about 20wt.%.

In the present application, the concentration of the stabilizer is about 10-200g/L. For example, the concentration of the stabilizer may be about 20-160g/L. For example, the concentration of the stabilizer may be about 20-100g/L. For example, the concentration of the stabilizer may be about 40-120g/L. For example, the concentration of the stabilizer can be about 10g/L, about 20g/L, about 30g/L, about 40g/L, about 50g/L, about 60g/L, about 70g/L, about 80g/L, about 90g/L, about 100g/L, about 110g/L, about 120g/L, about 130g/L, about 140g/L, about 150g/L, about 160g/L, about 170g/L, about 180g/L, about 190g/L, or about 200g/L.

### Surfactant

In the present application, the pharmaceutical composition may further comprise a surfactant. The surfactant may be a pharmaceutical excipient used to protect the protein (such as the IL-17A antibody) from various stress conditions, such as stirring, shearing, exposure to high temperature. The surfactant can also act as a solubilizer or prevent the protein from aggregating during the shaking process, and from being adsorbed or precipitated when in contact with the inner packaging material, air, or rubber plug.

In the pharmaceutical composition of the present application, the surfactant includes (but is not limited to): polyoxyethylene sorbitan fatty acid ester, polyoxyethylene hydrogenated castor oil, glycerol fatty acid ester, polysorbate, poloxamer, or a combination thereof.

For example, the polysorbate includes (but is not limited to): polysorbate 20 (PS-20, Tween-20), polysorbate 40 (PS-40), polysorbate 60 (PS-60), and polysorbate 80 (PS-80).

For example, the poloxamer includes (but is not limited to): poloxamer 188, poloxamer 108, poloxamer 124, or a combination thereof.

In some specific embodiments, the surfactant is polysorbate.

In the present application, the content of the surfactant is about 0.001-0.2 wt.%. For example, the content of the surfactant may be about 0.001-0.1 wt.%. For example, the content of the surfactant is about 0.005-0.05 wt.%, based on the total weight of the pharmaceutical composition. For example, the content of the surfactant is about 0.001%-0.1% (w/w), about 0.001%-0.09% (w/w), about 0.001%-0.08% (w/w), about 0.001%-0.07% (w/w), about 0.001%-0.06% (w/w), about 0.001%-0.05% (w/w), about 0.002%-0.04% (w/w), about 0.003%-0.03% (w/w), about 0.004%-0.025% (w/w), about 0.001%-0.045% (w/w), about 0.001%-0.04% (w/w), about 0.001%-0.035% (w/w), about 0.001%-0.03% (w/w), about 0.001%-0.12% (w/w), about 0.001%-0.13% (w/w), about 0.001%-0.15% (w/w), about 0.001%-0.16% (w/w), about 0.001%-0.17% (w/w), about 0.001%-0.18% (w/w), about 0.001%-0.19% (w/w) or about 0.001%-0.2% (w/w).

### Other pharmaceutically acceptable carriers

In the present application, the pharmaceutically acceptable carrier also includes a chelator. Chelators generally refer to multifunctional molecules with many negatively charged ligands and/or multi-electron ligands that can multivalently chelate metal ions with different affinities. Suitable multi-electron functional groups include carboxyl, hydroxyl and amino. Examples of chelator include aminopolycarboxylic acids such as ethylene diamine tetraacetic acid (EDTA), diethylene triamine pentaacetic acid (DTPA), nitrilotriacetic acid (NTA), N-2-acetamido-2-iminodiacetic acid (ADA), di(aminoethyl)ethylene glycol ether-N,N,N',N'-tetraacetic acid (EGTA), trans-cyclohexanediamine tetraacetic acid (DCTA), glutamic acid and/or aspartic acid, hydroxyaminocarboxylic acids such as N-hydroxyethyliminodiacetic acid (HIMDA), N,N-di-hydroxyethylglycine (N-di(hydroxyethyl)glycine) and/or N-(trihydroxymethylmethyl)glycine (N-tri(hydroxymethyl)methylglycine), and N-substituted glycines such as N-glycylglycine. Other candidate chelators may include 2-(2-amino-2-oxyethyl)aminoethanesulfonic acid (BES) and deferoxamine (DEF).

In the present application, the chelator may include, but is not limited to, disodium ethylene diamine tetraacetic acid (EDTA), diethylene triamine pentaacetic acid (DTPA), or a combination thereof.

In that present application, the content of the chelator may be about 0.001- 0.01 wt.%, based on the total weight of the pharmaceutical composition. For example, the content of the chelator is about 0.001%-0.1% (w/w), about 0.001%-0.09% (w/w), about 0.001%-0.08% (w/w), about 0.001%-0.07% (w/w), about 0.001%-0.06% (w/w), about 0.001%-0.05% (w/w), about 0.002%-0.04% (w/w), about 0.003%-0.03% (w/w), about 0.004%-0.025% (w/w), about 0.001%-0.045% (w/w), about 0.001%-0.04% (w/w), about 0.001%-0.035% (w/w), about 0.001%-0.03% (w/w), about 0.001%-0.12% (w/w), about 0.001%-0.13% (w/w), about 0.001%-0.15% (w/w), about 0.001%-0.16% (w/w), about 0.001%-0.17% (w/w), about 0.001%-0.18% (w/w), about 0.001%-0.19% (w/w) or about 0.001%-0.2% (w/w).

For example, the pharmaceutical composition may comprise the following substances: 20mM sodium acetate-acetic acid buffer, 7 wt.% sucrose, 0.03 wt.% Tween-80, pH 5.2±0.3, and humanized anti-human IL-17A monoclonal antibody of 150mg/mL.

For example, the pharmaceutical composition may comprise the following substances: 20mM sodium acetate-acetic acid buffer, 7 wt.% trehalose, 0.03 wt.% Tween-80, pH 5.2±0.3, and humanized anti-human IL-17A monoclonal antibody of 150mg/mL.

For example, the pharmaceutical composition may comprise the following substances:20mM histidine-acetic acid buffer, 7 wt.% sucrose, 0.03 wt.% Tween-80, pH5.3±0.3, and humanized anti-human IL-17A monoclonal antibody of 150mg/mL.

For example, the pharmaceutical composition may comprise the following substances: 20mM histidine-acetic acid buffer, 7 wt.% sucrose, 0.03 wt.% Tween-80, pH5.6±0.3, and humanized anti-human IL-17A monoclonal antibody of 150mg/mL.

For example, the pharmaceutical composition may comprise the following substances: 40mM histidine-histidine hydrochloride buffer, 1 wt.% proline, 4.5 wt.% sucrose, 0.03 wt.% Tween-80, pH5.8±0.3, and humanized anti-human IL-17A monoclonal antibody of 150mg/mL.

For example, the pharmaceutical composition may comprise the following substances: 20mM histidine-acetic acid buffer, 7.5 wt.% sucrose, 0.03 wt.% Tween-80, pH5.4±0.3, and humanized anti-human IL-17A monoclonal antibody of 150mg/mL.

For example, the pharmaceutical composition may comprise the following substances: 20mM sodium acetate-acetic acid buffer, 8 wt.% sucrose, 0.01 wt.% Tween-80, pH5.7±0.3, and humanized anti-human IL-17A monoclonal antibody of 150mg/mL.

In the present application, the stability of the antibody formulation can be evaluated by measuring the content of the target protein, fragment or aggregate, or the particle size of the insolubles in the formulation, for example, by size exclusion high performance liquid chromatography (SE-HPLC), reducing capillary electrophoresis sodium dodecyl sulphate (rCE-SDS), non-reducing capillary electrophoresis sodium dodecyl sulphate (nrCE-SDS), imaged capillary isoelectric focusing (icIEF) and/or particle size (PM) detection technology.

### Lyophilized formulation, kit

The present application further provides a lyophilized formulation, which is prepared by the following method: freeze-drying the pharmaceutical composition of the present application to obtain the lyophilized formulation.

In the present application, the lyophilized formulation may further comprise a lyoprotectant.

In the present application, the lyoprotectant may be selected from the group consisting of sucrose, mannitol, trehalose, maltose, sorbitol, glucose, fructose, galactose, or a combination thereof.

In another aspect, the present application further provides a kit comprising the pharmaceutical composition, or the lyophilized formulation, and a container for containing the pharmaceutical composition or the lyophilized formulation.

In some embodiments, the kit further comprises instructions for use of the pharmaceutical composition or the lyophilized formulation.

### Use

In another aspect, the present application further provides use of the pharmaceutical composition, the lyophilized formulation or the kit in the preparation of a medicament for preventing and/or treating IL-17A-mediated diseases and/or disorders.

In the present application, the IL-17A-mediated diseases and/or disorders may include autoimmune diseases.

In another aspect, the present application further provides use of the pharmaceutical composition, the lyophilized formulation or the kit in the preparation of a medicament for preventing and/or treating autoimmune diseases and/or disorders.

In the present application, the disease and/or disorder may be mediated by IL-17A.

In some embodiments, the autoimmune disease is selected from the group consisting of psoriasis, arthritis, multiple sclerosis, ankylosing spondylitis, inflammatory bowel disease, systemic lupus erythematosus, type I diabetes, or a combination thereof.

In the present application, the arthritis may be rheumatoid arthritis.

In another aspect, the present application further provides a method for preventing and/or treating IL-17A-mediated diseases and/or disorders, comprising administering the pharmaceutical composition, the lyophilized formulation or the kit to a subject in need thereof.

In the present application, the IL-17A-mediated diseases and/or disorders include autoimmune diseases.

In the present application, the autoimmune disease can be selected from the group consisting of psoriasis, arthritis, multiple sclerosis, ankylosing spondylitis, inflammatory bowel disease, systemic lupus erythematosus, type I diabetes, or a combination thereof.

In another aspect, the present application further provides the pharmaceutical composition, the lyophilized formulation or the kit for use in preventing and/or treating IL-17A-mediated diseases and/or disorders.

In the present application, the IL-17A-mediated diseases and/or disorders include autoimmune diseases.

In the present application, the autoimmune disease can be selected from the group consisting of psoriasis, arthritis, multiple sclerosis, ankylosing spondylitis, inflammatory bowel disease, systemic lupus erythematosus, type I diabetes, or a combination thereof.

In the present application, the subject may be a human or a non-human mammal.

Without wishing to be bound by any theory, the following examples are only for illustrating the various technical solutions of the present application, and are not used to limit the scope of the invention of the present application.

### Examples

### General test methods

SEC-UPLC: The test is carried out by ACQUITY UPLC ^{®} Protein BEH SEC column (4.6mm × 150mm) and Waters ultra-high performance liquid chromatograph (Acquity H Class) with reference to "Pharmacopeia of the People's Republic of China" (2015, Part III) 0514 General Principles of High Performance Liquid Chromatography, and the purity is calculated by the area normalization method.

CEX-HPLC: The test is carried out by ProPac^{™} WCX-10 column (4mm × 250mm) and Waters high performance liquid chromatograph (E2695) with reference to "Pharmacopeia of the People's Republic of China" (2015, Part III) General Principles of High Performance Liquid Chromatography, and the purity is calculated by the area normalization method.

Determination of Tm (IF, full spectrum fluorescence): Using Uncle (Unchained Labs), heating is carried out at 0.3°C/min in the range of 25°C to 95°C, and the melting temperature Tm of different formulas is calculated using the full spectrum fluorescence module.

Determination of average particle size distribution (DLS, dynamic light scattering): The average particle size (Z-average) of different formulas at 25°C is determined by using the dynamic light scattering module of the Uncle instrument.

Determination of Tagg (SLS, static light scattering): Using Uncle instrument at 25°C to 95°C, heating is carried out at 0.3°C/min, and the initial aggregation temperature Tagg of antibody molecules of different formulas is calculated by using the static light scattering module.

Injection thrust test: The test is carried out at a speed of 200mm/min by using a medical package tear force tester.

Viscosity test: The test is carried out at 4°C to 80°C by using a Rheosense viscometer. The sample temperature is adjusted by the Thermocbe unit (temperature control unit), while the sample viscosity at the corresponding temperature is tested by using the m-VROC unit (viscosity test unit).

### Example 1 Suitable pH development

The humanized anti-human IL-17A monoclonal antibody (the amino acid sequence of HCDR1 is set forth in SEQ ID NO: 1, the amino acid sequence of HCDR2 is set forth in SEQ ID NO: 2, the amino acid sequence of HCDR3 is set forth in SEQ ID NO: 3, the amino acid sequence of the heavy chain variable region VH is set forth in SEQ ID NO: 7, the amino acid sequence of LCDR1 is set forth in SEQ ID NO: 4, the amino acid sequence of LCDR2 is set forth in SEQ ID NO: 5 (KVS), the amino acid sequence of LCDR3 is set forth in SEQ ID NO: 6, the amino acid sequence of the light chain variable region VL is set forth in SEQ ID NO: 8, the amino acid sequence of the heavy chain constant region is set forth in SEQ ID NO: 9, and the amino acid sequence of the light chain constant region is set forth in SEQ ID NO: 10) was replaced into acetate-acetic acid buffer with 8 wt.% sucrose and different pH values and histidine-acetic acid buffer with 8 wt.% sucrose and different pH values using the 30KD ultrafiltration membrane cassette (PLCTK) from Merk. The antibody concentration was adjusted to about 150mg/mL, and the antibody was aseptically dispensed into pre-filled syringes (1.0 mL/syringe) to obtain samples. (Note: The solvent of all the above buffers is sterile water for injection)

The newly prepared samples were subjected to injection thrust test using a medical package tear force tester at a test speed of 200 mm/min and a test temperature of 5±3°C. At the same time, the flow state of the sample placed inverted was observed.

**Table 1 Injection thrust and flow state of samples (150mg/mL) at different pH values**

| Sample name | Protein content (mg/mL) | Sample pH | Injection thrust (N) | | | Flow state |
|---|---|---|---|---|---|---|
| | | | Initial force | Maximum force | Average force | Real-time observation when kept inverted |
| Water | / | / | 3.7 | 2.1 | 2.0 | Flow normally |
| 20mM sodium acetate-acetic acid, with 13 wt.% sucrose, antibody of 150mg/mL. | 154 | 4.9 | 38.3 | 38.3 | 37.5 | Flow normally |
| | 153 | 5.1 | 38.1 | 37.6 | 37.0 | Flow normally |
| | 148 | 5.3 | 38.4 | 38.3 | 37.6 | Flow normally |
| | 153 | 5.4 | 40.7 | 38.4 | 37.8 | Flow normally |
| | 148 | 5.6 | 47.6 | 47.1 | 46.3 | Slightly slow flow rate |
| 20mM histidine-acetic acid, with 13 wt.% sucrose, antibody of 150mg/mL. | 152 | 5.1 | 34.9 | 33.8 | 33.1 | Flow normally |
| | 153 | 5.2 | 38.9 | 38.5 | 37.8 | Flow normally |
| | 153 | 5.4 | 32.9 | 32.1 | 31.5 | Flow normally |
| | 153 | 5.7 | 36.6 | 35.8 | 35.3 | Flow normally |
| | 154 | 5.9 | 52.5 | 52.0 | 51.3 | No flow |

As can be seen from Table 1, the 150 mg/mL sample has a good flow state in the lower pH range (4.9-5.6), and the corresponding injection thrust is also lower than that in the higher pH range (5.6-5.9).

### Example 2 High-concentration (150mg/mL) formulation formula screening

Humanized anti-human IL-17A monoclonal antibody pharmaceutical formulations were prepared according to the following formulas for stress, accelerated and long-term stability studies. The formula information is as follows:
Formula 1: 20mM sodium acetate-acetic acid buffer, 7 wt.% trehalose, 0.03 wt.% Tween-80, pH5.2, humanized anti-human IL-17A monoclonal antibody of 150mg/mL
Formula 2: 20mM histidine-acetic acid buffer, 7 wt.% sucrose, 0.03 wt.% Tween-80, pH5.3, humanized anti-human IL-17A monoclonal antibody of 150mg/mL
Formula 3: 20mM histidine-acetic acid buffer, 7 wt.% sucrose, 0.03 wt.% Tween-80, pH5.6, humanized anti-human IL-17A monoclonal antibody of 150mg/mL
Formula 4: 40mM histidine-histidine hydrochloride buffer, 1 wt.% proline, 4.5 wt.% sucrose, 0.03 wt.% Tween-80, pH5.8, humanized anti-human IL-17A monoclonal antibody of 150mg/mL

Note: The solvent of all the above buffers is sterile water for injection.

### Preparation method:

Formula 1: Firstly, the humanized anti-human IL-17A monoclonal antibodies (SEQ ID NO.: 7 and SEQ ID NO.: 8) were replaced into acetate-acetic acid buffer containing trehalose by using the 30KD ultrafiltration membrane cassette (PLCTK) from Merk, Tween-80 was added thereto, the antibody concentration was adjusted to about 150mg/mL by sodium acetate-acetic acid buffer, and the antibody was aseptically dispensed into pre-filled syringes (1.0mL/syringe) to obtain the final liquid formulation. The pH of the liquid formulation was determined to be 5.2.

Formula 2: Firstly, the humanized anti-human IL-17A monoclonal antibodies (SEQ ID NO.: 7 and SEQ ID NO.: 8) were replaced into histidine-acetic acid buffer containing sucrose by using the 30KD ultrafiltration membrane cassette (PLCTK) from Merk, Tween-80 was added thereto, the antibody concentration was adjusted to about 150mg/mL by histidine-acetic acid buffer, and the antibody was sterilized and filtered, and then aseptically dispensed into pre-filled syringes (1mL/syringe) to obtain the final liquid formulation. The pH of the liquid formulation was determined to be 5.3.

Formula 3: Firstly, the humanized anti-human IL-17A monoclonal antibodies (SEQ ID NO.: 7 and SEQ ID NO.: 8) were replaced into histidine-acetic acid buffer containing sucrose by using the 30KD ultrafiltration membrane cassette (PLCTK) from Merk, Tween-80 was added thereto, the antibody concentration was adjusted to about 150mg/mL by histidine-acetic acid buffer, and the antibody was sterilized and filtered, and then aseptically dispensed into pre-filled syringes (1mL/syringe) to obtain the final liquid formulation. The pH of the liquid formulation was determined to be 5.6.

Formula 4: Firstly, the humanized anti-human IL-17A monoclonal antibodies (SEQ ID NO.: 7 and SEQ ID NO.: 8) were replaced into histidine-histidine hydrochloride buffer containing sucrose and proline by using the 30KD ultrafiltration membrane cassette (PLCTK) from Merk, Tween-80 was added thereto, the antibody concentration was adjusted to about 150mg/mL by histidine-histidine hydrochloride buffer, and the antibody was sterilized and filtered, and then aseptically dispensed into pre-filled syringes (1mL/syringe) to obtain the final liquid formulation. The pH of the liquid formulation was determined to be 5.8.

### 2.1 Stress stability

The stress conditions include: shaking at room temperature for 3 days (200rmp), repeated freezing-thawing (5 cycles, freezing-thawing at -80°C±15°C/room temperature), etc. The samples were stored under various stress conditions (as shown in Table 6). Samples were taken for testing at specified time points or after specified treatment times. Their stability was evaluated by appearance, number of insoluble particles and purity. Some of the test results are shown in the table below.

**Table 2 Stress stability data**

| Sample name | Stress conditions | Appearance | Insoluble particles | | Purity | |
|---|---|---|---|---|---|---|
| | | | ≥10µm (particles/mL) | ≥25µm (particles/mL) | SEC purity | CEX main peak |
| Formula 1 | T0 control | Clear | 0 | 0 | 98.7% | 85.0% |
| | Repeated freezing-thawing for five times | Clear | / | / | 98.6% | / |
| | Shaking at room temperature for 3 days | Clear | 100 | 10 | 98.4% | 84.5% |
| Formula 2 | T0 control | Clear | 190 | 50 | 98.8% | 84.9% |
| | Repeated freezing-thawing for five times | Clear | / | / | 98.7% | / |
| | Shaking at room temperature for 3 days | Clear | 0 | 0 | 98.5% | 81.6% |
| Formula 3 | T0 control | Clear | 10 | 10 | 98.7% | 85.4% |
| | Repeated freezing-thawing for five times | Clear | / | / | 98.7% | / |
| | Shaking at room temperature for 3 days | Clear | 200 | 0 | 98.5% | 82.8% |
| Formula 4 | T0 control | Clear | 40 | 0 | 98.7% | 85.7% |
| | Repeated freezing-thawing for five times | Clear | / | / | 98.7% | / |
| | Shaking at room temperature for 3 days | Clear | 60 | 0 | 98.6% | 85.0% |

| | | | | | | |
|---|---|---|---|---|---|---|
| Note: T0 control is the control when preparation of the formulation is completed. | | | | | | |

As can be seen from Table 2, the stress conditions have little effect on the particles and purity of several formulas. The pharmaceutical compositions of formulas 1-4 are highly stable and can effectively protect the main drug component monoclonal antibody, thereby effectively resisting the destructive effects of various stress conditions.

### 2.2 Accelerated stability

The samples were stored under accelerated conditions (25°C±2°C), and samples were taken for testing at specified time points. Their stability was evaluated by appearance and purity. Some of the test results are shown in Table 3 below.

**Table 3 Summary of accelerated stability data**

| Sample name | Conditions | Appearance | Purity | |
|---|---|---|---|---|
| | | | SEC main peak | CEX main peak |
| Formula 1 | T0 control | Clear | 98.7% | 85.0% |
| | 1 month | Clear | 97.8% | 82.5% |
| | 2 months | Clear | 96.9% | 77.3% |
| | 3 months | Clear | 95.6% | 75.5% |
| Formula 2 | T0 control | Clear | 98.8% | 84.9% |
| | 1 month | Clear | 97.9% | 82.8% |
| | 2 months | Clear | 97.2% | 77.2% |
| | 3 months | Clear | 96.0% | 76.3% |
| Formula 3 | T0 control | Clear | 98.7% | 85.4% |
| | 1 month | Clear | 98.2% | 83.2% |
| | 2 months | Clear | 97.7% | 78.9% |
| | 3 months | Clear | 96.6% | 77.0% |
| Formula 4 | T0 control | Clear | 98.7% | 85.7% |
| | 1 month | Clear | 98.2% | 83.6% |
| | 2 months | Clear | 97.7% | 79.8% |
| | 3 months | Clear | 96.6% | 78.1% |

| | | | | |
|---|---|---|---|---|
| Note: T0 control is the control when preparation of the formulation is completed. | | | | |

It can be seen from Table 3 that the accelerated conditions will cause a certain degree of decline in the quality of the pharmaceutical formulation. During the entire accelerated test period, compared with the T0 samples, the SEC molecular sieve purity and CEX main peak content of the samples accelerated for 3 months of each formula decrease but still meet the quality standards; in short, the pharmaceutical compositions of several groups of formulas in the present application are very stable and can be stably stored for at least 3 months under accelerated conditions of 25°C.

### 2.3 Long-term stability

The samples were stored under long-term cold storage conditions (5°C±3°C), and samples were taken for testing at specified time points. Their stability was evaluated by appearance and purity. Some of the test results are shown in Table 4 below.

**Table 4 Long-term cold storage stability data**

| Sample name | Conditions | Appearance | Purity | |
|---|---|---|---|---|
| | | | SEC main peak | CEX main peak |
| Formula 1 | T0 control | Clear | 98.7% | 85.0% |
| | 3 months | Clear | 98.1% | 84.9% |
| Formula 2 | T0 control | Clear | 98.8% | 84.9% |
| | 3 months | Clear | 98.3% | 84.4% |
| Formula 3 | T0 control | Clear | 98.7% | 85.4% |
| | 3 months | Clear | 98.4% | 85.2% |
| Formula 4 | T0 control | Clear | 98.7% | 85.7% |
| | 3 months | Clear | 98.1% | 85.9% |

| | | | | |
|---|---|---|---|---|
| Note: T0 control is the control when preparation of the formulation is completed. | | | | |

As can be seen from Table 4, under long-term cod storage conditions (5°C±3°C), the pharmaceutical formulations have good stability, and the purity data of the three-month samples in each formula have no significant changes. In conclusion, these pharmaceutical compositions are highly stable and can be stored stably under long-term cold storage conditions.

In summary, it can be seen that in the several groups of humanized anti-human IL-17A monoclonal antibody pharmaceutical compositions prepared in Example 2, the humanized anti-human IL-17A monoclonal antibody maintains good stability under various stress conditions (high temperature, repeated freezing-thawing, and shaking), and can be stably stored for at least three months under accelerated conditions (25°C±2°C). Also, it shows high stability under long-term cold storage conditions and can be stably stored.

### Example 3 Evaluation of stability and injectability of preferred high-concentration (150mg/mL) formulation formulas

In order to comprehensively compare the stability and injectability of the high-concentration (150mg/mL) formulation formulas of humanized anti-human IL-17A monoclonal antibody drug with those of the early developed formulation formula (formula: 20mM sodium acetate-acetic acid buffer, 8 wt.% sucrose, 0.01 wt.% Tween-80, pH 5.7), we compared the formulation performance of the early formula at different antibody concentrations (100mg/mL and 150mg/mL) (formula 5, formula 6) and two preferred formulas of 150mg/mL (formula 7, formula 8). The specific information of the four groups of formulas is as follows:
Formula 5: 20mM sodium acetate-acetic acid buffer, 8 wt.% trehalose, 0.01 wt.% Tween-80, pH5.7, humanized anti-human IL-17A monoclonal antibody of 100mg/mL;
Formula 6: 20mM sodium acetate-acetic acid buffer, 8 wt.% trehalose, 0.01 wt.% Tween-80, pH5.7, humanized anti-human IL-17A monoclonal antibody of 150mg/mL;
Formula 7: 20mM sodium acetate-acetic acid buffer, 7 wt.% trehalose, 0.03 wt.% Tween-80, pH5.2, humanized anti-human IL-17A monoclonal antibody of 150mg/mL;
Formula 8: 20mM histidine-acetic acid buffer, 7.5 wt.% sucrose, 0.03 wt.% Tween-80, pH5.4, humanized anti-human IL-17A monoclonal antibody of 150mg/mL;

### Preparation method:

Formula 5: Firstly, the humanized anti-human IL-17A monoclonal antibodies (SEQ ID NO.: 7 and SEQ ID NO.: 8) were replaced into acetate-acetic acid buffer containing sucrose by using the 30KD ultrafiltration membrane cassette (PLCTK) from Merk, Tween-80 was added thereto, the antibody concentration was adjusted to about 100mg/mL by sodium acetate-acetic acid buffer, and the antibody was aseptically dispensed into pre-filled syringes (1.0mL/syringe) to obtain the final liquid formulation. The pH of the liquid formulation was determined to be 5.7.

Formula 6: Firstly, the humanized anti-human IL-17A monoclonal antibodies (SEQ ID NO.: 7 and SEQ ID NO.: 8) were replaced into acetate-acetic acid buffer containing sucrose by using the 30KD ultrafiltration membrane cassette (PLCTK) from Merk, Tween-80 was added thereto, the antibody concentration was adjusted to about 150mg/mL by sodium acetate-acetic acid buffer, and the antibody was aseptically dispensed into pre-filled syringes (1.0mL/syringe) to obtain the final liquid formulation. The pH of the liquid formulation was determined to be 5.7.

Formula 7: Firstly, the humanized anti-human IL-17A monoclonal antibodies (SEQ ID NO.: 7 and SEQ ID NO.: 8) were replaced into acetate-acetic acid buffer containing sucrose by using the 30KD ultrafiltration membrane cassette (PLCTK) from Merk, Tween-80 was added thereto, the antibody concentration was adjusted to about 150mg/mL by sodium acetate-acetic acid buffer, and the antibody was aseptically dispensed into pre-filled syringes (1.0mL/syringe) to obtain the final liquid formulation. The pH of the liquid formulation was determined to be 5.2.

Formula 8: Firstly, the humanized anti-human IL-17A monoclonal antibodies (SEQ ID NO.: 7 and SEQ ID NO.: 8) were replaced into histidine-acetic acid buffer containing sucrose by using the 30KD ultrafiltration membrane cassette (PLCTK) from Merk, Tween-80 was added thereto, the antibody concentration was adjusted to about 150mg/mL by histidine-acetic acid buffer, and the antibody was aseptically dispensed into pre-filled syringes (1.0mL/syringe) to obtain the final liquid formulation. The pH of the liquid formulation was determined to be 5.4.

### 3.1 Flow state and viscosity performance of four high-concentration formula formulations

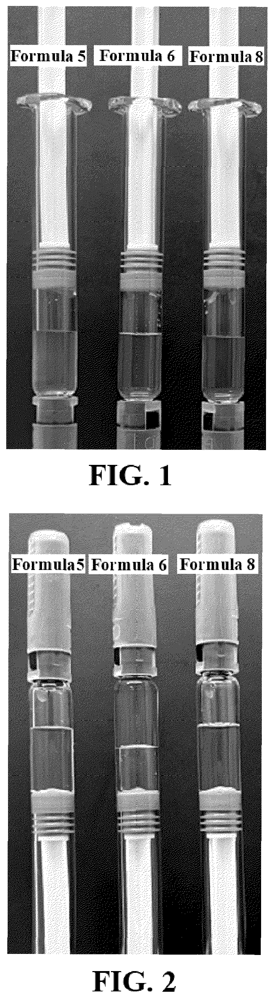

The humanized anti-human IL-17A monoclonal antibody pharmaceutical formulation samples prepared according to Example 3 were stored under long-term cold storage conditions (5°C±3°C), and the flow state of four groups of formulation samples when kept upright and inverted was photographed and compared. The upright view is shown in Fig. 1, and the inverted view is shown in Fig. 2.

The viscosity values at low temperature (4°C) and room temperature (23°C) were investigated. The viscosity of three groups of formulation samples was tested after being stored overnight under long-term cold storage conditions (5°C±3°C). The detection results are shown in Table 5 below.

**Table 5 Viscosity values of high-concentration formulation samples**

| Sample name | Temperature | Average viscosity (cP) |
|---|---|---|
| Formula 5 | Low temperature | 10.3 |
| | Room temperature | 4.4 |
| Formula 6 | Low temperature | 199.8 |
| | Room temperature | 12.1 |
| Formula 7 | Low temperature | 38.4 |
| | Room temperature | 14.3 |
| Formula 8 | Low temperature | 31.9 |
| | Room temperature | 11.3 |

As can be seen from Figs. 1 and 2, only formula 6 cannot flow when kept inverted at low temperature. Formulas 5 and 8 are flowable at low temperature.

It can be seen from Table 5 that the viscosity of formula 6 at low temperature (4°C) is 20 times that of formula 5 at low temperature (4°C). The viscosity of formula 8 at low temperature (4°C) is three times that of formula 5 at low temperature (4°C), and formula 8 is significantly superior to formula 6. Formula 8 is also slightly superior to formula 6 in terms of viscosity at room temperature (23°C). This indicates that the preferred formula formulation (formula 8) has better injectability at a high concentration (150mg/mL). Also, the viscosity of formula 7 at low temperature (4°C) is lower than that of formula 6 at low temperature (4°C), indicating that the injectability of the preferred formula formulation (formula 7) at a high concentration (150mg/mL) is better.

In summary, the formulations of the preferred formulas (formula 7, formula 8) have better flowability and lower viscosity than the formula 6 derived from the early formula (antibody concentration 150mg/mL). The injectability of the high-concentration formulation is significantly improved.

### 3.2 Stability of high-concentration formula formulations

The stability data of the four groups of formulas (formula 5, formula 6, formula 7, formula 8) are shown in Table 6.

**Table 6 Stability data of high-concentration formulation samples**

| Sample name | Conditions | Purity | |
|---|---|---|---|
| | | SEC main peak | CEX main peak |
| Formula 5 | T0 | 99.3% | 83.8% |
| | 25°C 1M | 98.9% | 78.9% |
| | 25°C 2M | 97.8% | 76.0% |
| | 25°C 3M | 97.4% | 73.4% |
| | 5°C 3M | 98.3% | 81.8% |
| | 5°C 6M | 97.4% | 83.7% |
| | 5°C 9M | 96.7% | 81.6% |
| | 5°C 12M | 97.7% | 81.6% |
| Formula 6 | T0 | 97.6% | 83.9% |
| | 25°C 1M | 92.3% | 80.9% |
| | 25°C 2M | 97.4% | 73.3% |
| | 25°C 3M | 96.8% | 68.2% |
| | 5°C 3M | 99.0% | 82.4% |
| | 5°C 6M | 98.5% | 82.0% |
| | 5°C 9M | 98.5% | 82.1% |
| | 5°C 12M | 98.8% | 79.5% |
| Formula 7 | T0 | 98.6% | 84.9% |
| | 25°C 1M | 97.6% | 82.1% |
| | 25°C 2M | 96.8% | 78.0% |
| | 25°C 3M | 95.5% | 75.6% |
| | 5°C 3M | 98.0% | 85.1% |
| | 5°C 7M | 98.1% | 85.9% |
| | 5°C 13M | 98.0% | 84.2% |
| Formula 8 | T0 | 99.7% | 87.9% |
| | 25°C 1M | 99.4% | 84.2% |
| | 25°C 3M | 98.8% | 75.6% |
| | 5°C 3M | 99.6% | 86.8% |
| | 5°C 6M | 99.5% | 85.8% |
| | 5°C 9M | 99.4% | 86.0% |

Table 6 show that the stability of formula 6 resulting from increasing the concentration of the early formula (150mg/mL) is worse than that of the early formula 5 (100mg/mL) (more reduction in the purity of CEX main peak at 5°C 12M and 25°C 3M), formula 7 (150mg/mL) is more stable than formula 5 (less reduction in the purity of SEC/CEX main peak at 5°C 13M, close performance at 25°C 3M), and formula 8 (150mg/mL) is more stable than formula 5 (at 5°C 9M and 25°C 3M, less reduction in the purity of SEC main peak and close CEX performance).

In order to meet the demand for increased clinical subcutaneous injection doses, the concentration of antibody drugs needs to be further increased. The study found that compared with the early formula 5 (100mg/mL), directly increasing the antibody concentration to 150mg/mL (formula 6) based on the early formula results in a significant decrease in stability and failure to flow at all at low temperature, which has an adverse effect on production and manufacture, drug appearance, administration process and shelf life. After systematic formula development and optimization, formula 7 (150mg/mL) and formula 8 (150mg/mL) completely alleviate a variety of problems found in formula 6, and have been ahead of the early formula 5 (100mg/mL) in many aspects such as stability and drug appearance. In summary, the excellent performance of high-concentration preferred formulation formulas can better support clinical development, and improve the competitiveness of drugs after marketing.

The foregoing details are provided by way of explanation and illustration and are not intended to limit the scope of the attached claims. Various modifications to the embodiments according to the present application will be apparent to those of ordinary skill in the art and remain within the scope of the appended claims and their equivalents.

## Claims

1. A pharmaceutical composition, comprising: an IL-17A antibody, and optionally a pharmaceutically acceptable carrier, wherein the concentration of the IL-17A antibody in the pharmaceutical composition is 125-155 mg/mL.

2. The pharmaceutical composition according to claim 1, wherein the IL-17A antibody is an anti-human IL-17A antibody.

3. The pharmaceutical composition according to claim 2, wherein the anti-human IL-17A antibody is a humanized anti-human IL-17A antibody.

4. The pharmaceutical composition according to any one of claims 1 to 3, wherein the IL-17A antibody comprises three complementary determining regions HCDR1, HCDR2 and HCDR3 of an antibody heavy chain variable region and three complementary determining regions LCDR1, LCDR2 and LCDR3 of an antibody light chain variable region, the HCDR1 comprises an amino acid sequence as set forth in SEQ ID NO: 1, the HCDR2 comprises an amino acid sequence as set forth in SEQ ID NO: 2, the HCDR3 comprises an amino acid sequence as set forth in SEQ ID NO: 3, the LCDR1 comprises an amino acid sequence as set forth in SEQ ID NO: 4, the LCDR2 comprises an amino acid sequence as set forth in SEQ ID NO: 5 (KVS), and the LCDR3 comprises an amino acid sequence as set forth in SEQ ID NO: 6.

5. The pharmaceutical composition according to any one of claims 1 to 4, wherein the heavy chain variable region VH of the IL-17A antibody comprises an amino acid sequence as set forth in SEQ ID NO: 7, and the light chain variable region of the IL-17A antibody comprises an amino acid sequence as set forth in SEQ ID NO: 8.

6. The pharmaceutical composition according to any one of claims 1 to 5, wherein the concentration of the IL-17A antibody is 130 mg/mL.

7. The pharmaceutical composition according to any one of claims 1 to 5, wherein the concentration of the IL-17A antibody is 150 mg/mL.

8. The pharmaceutical composition according to any one of claims 1 to 7, wherein the dosage form of the pharmaceutical composition is a liquid formulation.

9. The pharmaceutical composition according to any one of claims 1 to 7, wherein the dosage form of the pharmaceutical composition is an injection formulation or an infusion formulation.

10. The pharmaceutical composition according to any one of the claims 1 to 9, wherein the pharmaceutically acceptable carrier comprises a buffer, a stabilizer and/or a surfactant.

11. The pharmaceutical composition according to claim 10, wherein the buffer is selected from the group consisting of acetate-acetic acid buffer, citrate-citric acid buffer, phosphate buffer, histidine-histidine hydrochloride buffer, histidine-acetic acid buffer, tris(hydroxymethyl)aminomethane (Tris)-hydrochloride buffer, phosphoric acid-citrate salt, or a combination thereof.

12. The pharmaceutical composition according to any one of claims 10 to 11, wherein the buffer is selected from the group consisting of acetate-acetic acid buffer, histidine-histidine hydrochloride buffer, histidine-acetic acid buffer, tris(hydroxymethyl)aminomethane (Tris)-hydrochloride buffer, phosphoric acid-citrate salt, or a combination thereof.

13. The pharmaceutical composition according to any one of claims 10 to 12, wherein the concentration of the buffer is 5-100mM.

14. The pharmaceutical composition according to any one of claims 10 to 13, wherein the concentration of the buffer is 10-80mM.

15. The pharmaceutical composition according to any one of claims 10 to 14, wherein the concentration of the buffer is 10-50mM.

16. The pharmaceutical composition according to any one of claims 10 to 15, wherein the solvent of the buffer is water.

17. The pharmaceutical composition according to any one of claims 10 to 16, wherein the stabilizer is selected from the group consisting of sodium chloride, amino acid, sugar alcohol, or a combination thereof.

18. The pharmaceutical composition according to claim 17, wherein the amino acid is one or more selected from the group consisting of proline, arginine, glycine, histidine, methionine, or a combination thereof.

19. The pharmaceutical composition according to any one of claims 17 to 18, wherein the sugar alcohol is selected from the group consisting of sucrose, mannitol, trehalose, maltose, sorbitol, or a combination thereof.

20. The pharmaceutical composition according to any one of claims 17 to 19, wherein the stabilizer is proline, histidine, arginine, sucrose, trehalose, mannitol, or a combination thereof.

21. The pharmaceutical composition according to any one of claims 17 to 20, wherein the concentration of the sodium chloride is 50-400mM.

22. The pharmaceutical composition according to any one of claims 17 to 21, wherein the concentration of the sodium chloride is 100-300mM.

23. The pharmaceutical composition according to any one of claims 17 to 22, wherein the concentration of the sodium chloride is 150-300mM.

24. The pharmaceutical composition according to any one of claims 17 to 23, wherein the concentration of the amino acid is 10-300mM.

25. The pharmaceutical composition according to any one of claims 17 to 24, wherein the concentration of the amino acid is 30-180mM.

26. The pharmaceutical composition according to any one of claims 17 to 25, wherein the concentration of the amino acid is 50-150mM.

27. The pharmaceutical composition according to any one of claims 17 to 26, wherein the concentration of the sugar alcohol is 0.1-20 wt.%, based on the total weight of the pharmaceutical composition.

28. The pharmaceutical composition according to any one of claims 17 to 27, wherein the concentration of the sugar alcohol is 2-16 wt.%, based on the total weight of the pharmaceutical composition.

29. The pharmaceutical composition according to any one of claims 17 to 28, wherein the concentration of the sugar alcohol is 2-14 wt.%, based on the total weight of the pharmaceutical composition.

30. The pharmaceutical composition according to any one of claims 17 to 29, wherein the concentration of the sugar alcohol is 4-12 wt.%, based on the total weight of the pharmaceutical composition.

31. The pharmaceutical composition according to any one of claims 10 to 30, wherein the content of the stabilizer is 0.1-20 wt.%, based on the total weight of the pharmaceutical composition.

32. The pharmaceutical composition according to any one of claims 10 to 31, wherein the content of the stabilizer is 2-16 wt.%, based on the total weight of the pharmaceutical composition.

33. The pharmaceutical composition according to any one of claims 10 to 32, wherein the content of the stabilizer is 2-14 wt.%, based on the total weight of the pharmaceutical composition.

34. The pharmaceutical composition according to any one of claims 10 to 33, wherein the content of the stabilizer is 4-12 wt.%, based on the total weight of the pharmaceutical composition.

35. The pharmaceutical composition according to any one of claims 10 to 34, wherein the concentration of the stabilizer is 10-200g/L.

36. The pharmaceutical composition according to any one of claims 10 to 35, wherein the concentration of the stabilizer is 20-160g/L.

37. The pharmaceutical composition according to any one of claims 10 to 36, wherein the concentration of the stabilizer is 20-140g/L.

38. The pharmaceutical composition according to any one of claims 10 to 37, wherein the concentration of the stabilizer is 40-120g/L.

39. The pharmaceutical composition according to any one of claims 10 to 38, wherein the surfactant is selected from the group consisting of polyoxyethylene sorbitan fatty acid ester, polyoxyethylene hydrogenated castor oil, glycerol fatty acid ester, polysorbate, poloxamer, or a combination thereof.

40. The pharmaceutical composition according to claim 39, wherein the polysorbate is selected from the group consisting of polysorbate 20 (PS-20, Tween-20), polysorbate 40 (PS-40), polysorbate 60 (PS-60), and polysorbate 80 (PS-80).

41. The pharmaceutical composition according to any one of claims 39 to 40, wherein the poloxamer is selected from the group consisting of poloxamer 188, poloxamer 108, poloxamer 124, or a combination thereof.

42. The pharmaceutical composition according to any one of claims 10 to 41, wherein the surfactant is polysorbate.

43. The pharmaceutical composition according to any one of claims 10 to 42, wherein the content of the surfactant is 0.001-0.2 wt.%, based on the total weight of the pharmaceutical composition.

44. The pharmaceutical composition according to any one of claims 10 to 43, wherein the content of the surfactant is 0.001-0.1 wt.%, based on the total weight of the pharmaceutical composition.

45. The pharmaceutical composition according to any one of claims 10 to 44, wherein the content of the surfactant is 0.005-0.05 wt.%, based on the total weight of the pharmaceutical composition.

46. The pharmaceutical composition according to any one of claims 1 to 45, wherein the pharmaceutically acceptable carrier further comprises a chelator.

47. The pharmaceutical composition according to claim 46, wherein the chelator is selected from the group consisting of disodium ethylene diamine tetraacetic acid (EDTA), diethylene triamine pentaacetic acid (DTPA), or a combination thereof.

48. The pharmaceutical composition according to any one of claims 46 to 47, wherein the content of the chelator is 0.001-0.01 wt.%, based on the total weight of the pharmaceutical composition.

49. The pharmaceutical composition according to any one of claims 1 to 48, wherein the pH range of the pharmaceutical composition is 4.5-7.2.

50. The pharmaceutical composition according to any one of claims 1 to 49, wherein the pH range of the pharmaceutical composition is 4.7-6.5.

51. The pharmaceutical composition according to any one of claims 1 to 50, wherein the pH range of the pharmaceutical composition is 4.7-6.0.

52. The pharmaceutical composition according to any one of claims 1 to 51, wherein the buffer in the pharmaceutical composition is acetate-acetic acid buffer, and the pharmaceutical composition has a pH of 4.7-6.0.

53. The pharmaceutical composition according to any one of claims 1 to 51, wherein the buffer in the pharmaceutical composition is histidine-acetic acid buffer, and the pharmaceutical composition has a pH of 4.7-6.0.

54. The pharmaceutical composition according to any one of claims 1 to 51, comprising 20mM sodium acetate-acetic acid buffer, 7 wt.% sucrose, 0.03 wt.% Tween-80, pH5.2±0.3, and anti-human IL-17A monoclonal antibody of 150mg/mL.

55. The pharmaceutical composition according to any one of claims 1 to 51, comprising 20mM histidine-acetic acid buffer, 7.5wt.% sucrose, 0.03 wt.% Tween-80, pH5.4±0.3, and anti-human IL-17A monoclonal antibody of 150mg/mL.

56. A lyophilized formulation, comprising the pharmaceutical composition according to any one of claims 1 to 55.

57. The lyophilized formulation according to claim 56, being prepared by the following method: freeze-drying the pharmaceutical composition according to any one of claims 1 to 55 to obtain the lyophilized formulation.

58. The lyophilized formulation according to any one of claims 56 to 57, further comprising a lyoprotectant.

59. The lyophilized formulation according to claim 58, wherein the lyoprotectant is selected from the group consisting of sucrose, mannitol, trehalose, maltose, sorbitol, glucose, fructose, galactose, or a combination thereof.

60. A kit, comprising the pharmaceutical composition according to any one of claims 1 to 55, or the lyophilized formulation according to any one of claims 56 to 59, and a container for containing the pharmaceutical composition or the lyophilized formulation.

61. The kit according to claim 60, further comprising instructions for use of the pharmaceutical composition or the lyophilized formulation.

62. Use of the pharmaceutical composition according to any one of claims 1 to 55, the lyophilized formulation according to any one of claims 56 to 59, or the kit according to any one of claims 60 to 61 in the preparation of a medicament for preventing and/or treating IL-17A-mediated diseases and/or disorders.

63. The use according to claim 62, wherein the IL-17A-mediated diseases and/or disorders include autoimmune diseases.

64. Use of the pharmaceutical composition according to any one of claims 1 to 55, the lyophilized formulation according to any one of claims 56 to 59, or the kit according to any one of claims 60 to 61 in the preparation of a medicament for preventing and/or treating autoimmune diseases and/or disorders.

65. The use according to claim 64, wherein the disease and/or disorder is mediated by IL-17A.

66. The use according to any one of claims 63 to 65, wherein the autoimmune disease is selected from the group consisting of psoriasis, arthritis, multiple sclerosis, ankylosing spondylitis, inflammatory bowel disease, systemic lupus erythematosus, type I diabetes, or a combination thereof.

67. The use according to claim 66, wherein the arthritis is rheumatoid arthritis.

68. A method for preventing and/or treating IL-17A-mediated diseases and/or disorders, comprising administering the pharmaceutical composition according to any one of claims 1 to 55, the lyophilized formulation according to any one of claims 56 to 59, or the kit according to any one of claims 60 to 61 to a subject in need thereof.

69. The method according to claim 68, wherein the IL-17A-mediated diseases and/or disorders include autoimmune diseases.

70. The method according to claim 69, wherein the autoimmune disease is selected from the group consisting of psoriasis, arthritis, multiple sclerosis, ankylosing spondylitis, inflammatory bowel disease, systemic lupus erythematosus, type I diabetes, or a combination thereof.

71. The pharmaceutical composition according to any one of claims 1 to 55, the lyophilized formulation according to any one of claims 56 to 59, or the kit according to any one of claims 60 to 61 for use in preventing and/or treating IL-17A-mediated diseases and/or disorders.

72. The pharmaceutical composition, the lyophilized formulation, or the kit according to claim 71, wherein the IL-17A-mediated diseases and/or disorders include autoimmune diseases.

73. The pharmaceutical composition, the lyophilized formulation, or the kit according to claim 72, wherein the autoimmune disease is selected from the group consisting of psoriasis, arthritis, multiple sclerosis, ankylosing spondylitis, inflammatory bowel disease, systemic lupus erythematosus, type I diabetes, or a combination thereof.
